(19)

**Europäisches Patentamt**
**European Patent Office**
**Office européen des brevets**

(11)  **EP 4 004 941 B1**

(12)  # EUROPEAN PATENT SPECIFICATION

(45) Date of publication and mention
of the grant of the patent:
**10.12.2025  Bulletin 2025/50**

(51) International Patent Classification (IPC):
*G16H 50/20* (2018.01)    *G16B 40/20* (2019.01)

(21) Application number: **20737059.4**

(52) Cooperative Patent Classification (CPC):
**G16B 40/20; G16H 50/20**

(22) Date of filing: **02.07.2020**

(86) International application number:
**PCT/IB2020/056251**

(87) International publication number:
**WO 2021/014243 (28.01.2021 Gazette 2021/04)**

(54) **A METHOD TO ASSIST IN THE EARLY DIAGNOSIS OF PANCREATIC ADENOCARCINOMA**

VERFAHREN ZUR UNTERSTÜTZUNG DER FRÜHEN DIAGNOSE VON
PANKREAS-ADENOKARZINOMEN

PROCÉDÉ POUR AIDER AU DIAGNOSTIC PRÉCOCE DE L'ADÉNOCARCINOME PANCRÉATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.07.2019  IT 201900012555**

(43) Date of publication of application:
**01.06.2022  Bulletin 2022/22**

(73) Proprietors:
• **Università degli Studi di Roma "La Sapienza"**
**00185 Roma (IT)**
• **Università Campus Bio-Medico di Roma**
**00128 Roma (IT)**

(72) Inventors:
• **CARACCIOLO, Giulio**
**00185 Roma (IT)**
• **POZZI, Daniela**
**00185 Roma (IT)**
• **PALCHETTI, Sara**
**00185 Roma (IT)**
• **DIGIACOMO, Luca**
**00185 Roma (IT)**
• **CAPUTO, Damiano**
**00128 Rome (IT)**
• **COPPOLA, Roberto**
**00128 Rome (IT)**

(74) Representative: **Predazzi, Valentina et al**
**Società Italiana Brevetti S.p.A.**
**Piazza di Pietra, 39**
**00186 Roma (IT)**

(56) References cited:
• **JEAN M. LEWIS ET AL: "Integrated Analysis of
Exosomal Protein Biomarkers on Alternating
Current Electrokinetic Chips Enables Rapid
Detection of Pancreatic Cancer in Patient
Blood", ACS NANO, vol. 12, no. 4, 23 March 2018
(2018-03-23), US, pages 3311 - 3320,
XP055678694, ISSN: 1936-0851, DOI: 10.1021/
acsnano.7b08199**
• **D. CAPUTO ET AL: "A protein corona-enabled
blood test for early cancer detection",
NANOSCALE, vol. 9, no. 1, 1 January 2017
(2017-01-01), United Kingdom, pages 349 - 354,
XP055678638, ISSN: 2040-3364, DOI: 10.1039/
C6NR05609A**
• **YAN TECK HO ET AL: "Protein Corona Formed
from Different Blood Plasma Proteins Affects the
Colloidal Stability of Nanoparticles Differently",
BIOCONJUGATE CHEMISTRY, vol. 29, no. 11, 22
October 2018 (2018-10-22), US, pages 3923 -
3934, XP055678878, ISSN: 1043-1802, DOI:
10.1021/acs.bioconjchem.8b00743**

**(Cont. next page)**

EP 4 004 941 B1

- XIAOHUA HUANG ET AL: "Gold Nanoparticle Based Platforms for Circulating Cancer Marker Detection", NANOTHERANOSTICS, vol. 1, no. 1, 1 January 2017 (2017-01-01), pages 80 - 102, XP055678689, ISSN: 2206-7418, DOI: 10.7150/ntno.18216
- GARCÍA VENCE MARÍA ET AL: "Potential clinical applications of the personalized, disease-specific protein corona on nanoparticles", CLINICA CHIMICA ACTA, ELSEVIER BV, AMSTERDAM, NL, vol. 501, 31 October 2019 (2019-10-31), pages 102 - 111, XP085994475, ISSN: 0009-8981, [retrieved on 20191031], DOI: 10.1016/J.CCA.2019.10.027
- LUCA DIGIACOMO ET AL: "The biomolecular corona of gold nanoparticles in a controlled microfluidic environment", LAB ON A CHIP, vol. 19, no. 15, 23 July 2019 (2019-07-23), pages 2557 - 2567, XP055678687, ISSN: 1473-0197, DOI: 10.1039/C9LC00341J

## Description

The present invention relates to a novel method for the early diagnosis of pancreatic adenocarcinoma, comprising the use of gold nanoparticles and a subsequent image analysis, a method of screening or monitoring pathological and non-pathological patients to identify pathological patients at risk of pancreatic adenocarcinoma, comprising the use of gold nanoparticles and a subsequent image analysis, a kit comprising reactants for the early diagnosis of pancreatic adenocarcinoma.

STATE OF THE ART

Pancreatic adenocarcinoma is a cancer burdened by fatal prognosis and with a progressively increasing incidence. The time pattern of the incidence of this neoplasia, without considering population age variations, is on the rise both in female and male individuals (+1% and +1.3%/year, respectively). A further increase of new cases of the disease is expected in the next years, from 12.180 of 2020 to 13.928 of 2030. The only possibility of treatment, with a 5-year survival of 15%-20%, is offered by the surgical removal of the disease. Surgical resection, however, unfortunately to date is implementable in only 20% of patients. At diagnosis, in fact, the surgical approach is precluded to about 80-95% of the subjects, due to the presence of local invasion of the vascular structures, or to concomitant presence of distant metastases. Pancreatic adenocarcinoma exhibits insidious behavior, frequently asymptomatic or with entirely non-specific symptoms in the early stages and demonstrates a rapid progression to more advanced stages.

However, it has been demonstrated that about 15 years are necessary for the sequence of genome alterations intervening between the tumor origin and its metastatization to be completed (Yachida S, Jones S, Bozic I, et al. Distant metastasis occurs late during the genetic evolution of pancreatic cancer. Nature. 2010; 467(7319):1114-7). This type of clinical history, along with biological features, make the disease highly lethal. From the foregoing, there emerges the absolute need to be able to identify the disease in an early stage transpires, yet unfortunately to date there are no methods to be used for the early diagnosis that be scarcely invasive and having high sensitivity and specificity. The lack of sensitive and specific biomarkers induced numerous experts to search for a molecular signature typing the tumor by proteomic profiling approach in mass spectrometry.

With the use of nanomaterials in biology and medicine, it has been discovered that nanoparticles, contacted with biological fluids, adsorb on their surfaces (depending on their size, shape and material of which they are made up), a protein layer, developing what is described as "protein crown" (or "protein corona").

It has recently been reported in the literature that nanoparticles (i.e., particles with at least one characteristic size ranging from 1 to 100 nm), after interaction with human plasma, coat themselves with a protein layer referred to as "protein crown". Crown (Corona) formation is a complex process in which a delicate balance between electrostatic, hydrophobic and van der Waals interactions regulates its final protein composition. Recent research demonstrate that protein crown composition mainly depends on three factors: i) the chemico-physical properties of the nanoparticles (e.g., size, charge, material, surface chemistry); ii) the protein composition of the biological fluid; iii) environmental factors (e.g., temperature and exposure time). It has been demonstrated that the composition of the "protein crown" does not mirror slavishly human proteome composition but depends on the affinity of each specific protein for the particle surface. It follows that proteins scarcely abundant in human blood, yet highly affine to the nanoparticle surface, can be far more enriched in the protein crown compared to proteins highly abundant in human blood (like, e.g., serum albumin), but scarcely affine to the nanoparticle surface. Recently, Harvard Medical School researchers have demonstrated that the protein crown (corona) forming on the nanoparticle surface after interaction with human plasma is "personalized" (Hajipour, Mohammad Javad, et al. "Personalized disease-specific protein corona influences the therapeutic impact of graphene oxide." Nanoscale 7.19 (2015): 8978-8994). This means that protein crown composition varies subjectwise as a result of individual variation in proteome composition. In fact, the analysis of changes in the proteome allows to identify proteins associated with the presence of a pathological condition, that may act as early-phase diagnostic markers, prognosis indicators or predictors of response to a treatment. Recently, many studies on the protein crown at physiological pH have become available, while little is still known on quali/quantitative crown modification under acidic conditions such as those encountered in inflamed tissues, tumor masses, lysosomes.

The non-patent document Colapicchioni, et al "Personalized liposome-protein corona in the blood of breast, gastric and pancreatic cancer patients " International Journal of Biochemistry and Cell Biology 2016, 75, 180-187 discloses that protein coronas isolated from the plasma of patients suffering from breast tumor, stomach tumor and pancreatic tumor are statistically different from each other. However, no image processing was carried out, nor any test able to predict a sample's belonging to the group of healthy patients or of the oncological patients was developed.

The non-patent document Caputo et al "A protein corona-enabled blood test for early cancer detection" Nanoscale, 2017, 9, 349-354 discloses the possibility to use the crown forming around anionic liposomes comprised of the dioleoyl-phosphatidyl-glycerol (DOPG) lipid to discriminate healthy subjects from patients suffering from pancreatic adenocarcinoma. Proof of feasibility as to the possibility of developing a blood test was provided.

In the state of the art, instead, a possible use of the protein crown depositing on nanoparticles for the early diagnosis of pancreatic adenocarcinoma is not known. Current attempts at developing screening tests for the early diagnosis of pancreatic adenocarcinoma have mainly focused on serum biomarkers. Ca 19.9 is the only marker approved by the Food and Drug Administration in clinical practice for pancreatic adenocarcinoma. However, CA19-9 use as screening tool for pancreatic cancer in the general population in unacceptable owing to its low sensitivity (median 79%, with a range of 70-90%) and specificity (median 82%, with a range of 68-91%).

Precisely with the aim of increasing the diagnostic accuracy of Ca 19.9, in the last few years various biomarker panels have been identified, which by combining various proteins thereamong, with or without Ca 19.9, proved useful in discriminating the subjects suffering from pancreatic adenocarcinoma compared to healthy controls or subjects suffering from other pathologies, such as chronic pancreatitis. Some of these panels proved also able to distinguish tumors in their initial stage, as compared to more advanced ones. Unfortunately, to date these methodologies are not applicable on a large scale in daily clinical practice, as they are the result of highly complex and costly technologies and require highly specialized personnel.

In comparison with serum markers other modes were taken into consideration, such as marker collection and study on pancreatic juice. For this purpose, a more costly and invasive procedure is needed, endoscopic retrograde cholangiopancreatography (ERCP) or endoscopic ultrasound-guided fine-needle aspiration (EUS-FNA). These techniques, and in particular ERCP, are, among other things, burdened by a nonnegligible rate of iatrogenic acute pancreatitis. Some biomarkers have also been identified by analyzing ductal brushing and cytology collections obtained by ERCP or FNA. However, in these cases as well, a sensitivity sufficiently high to justify the use of techniques so invasive and costly to diagnose early-stage pancreatic cancer was not reached.

Various imaging methods are used to identify neoplasias in patients that are symptomatic or with strongly suspected pancreatic neoplasia. The main imaging modes for pancreatic tumor detection are abdominal echography, echoendoscopy, CT, magnetic resonance, ERCP and positron-emission tomography (PET). Echography is non-invasive, easily available and does not expose the patient to ionizing radiations. However, owing to the position of the pancreas in the retroperitoneum, its accuracy is of <70%. Instead, transgastrically-performed echoendoscopy has a >95% sensitivity. Unfortunately, as with all echographic examinations, both are affected by the operator-related variability issue. CT with contrast media (cm) administration has a high sensitivity (90%) and specificity (99%), with a lower interobserver variability compared to echography. However, CT exposes patients to ionizing radiations and, due to the requirement of intravenous contrast, is not ideal for use in all patients, especially those with kidney failure or allergies. Magnetic resonance has a sensitivity and a specificity similar to CT, yet it has longer acquisition times and requires greater collaboration by patients, who have to keep still in order to obtain an accurate image. In general, high costs, invasiveness and the high rate of false positives represent the main drawbacks of the use of imaging techniques in screening programs of most tumors, and above all of pancreatic tumor.

SUMMARY OF THE INVENTION

Though to date there is an incomplete understanding of risk factors for pancreatic cancer, some groups have since years been identified as high-risk on the basis of clinical and genetic characteristics, and they might represent the target of application of the proposed technology. Clinical risk factors include age, obesity, smoking, diabetes and chronic pancreatitis. Pancreatic ductal adenocarcinoma appears in >95% of cases after 45 years of age, obese subjects have an up to 1.8-fold higher risk of developing the disease, as well as smokers and those who have quit smoking since less than 5 years, and those who have documented familiarity. A 1.76-fold higher risk is faced by diabetic patients, and new-onset diabetes can represent an early indicator of pancreatic neoplasia. In patients suffering from chronic pancreatitis the risk of tumor is more than doubled. Instead, as to genetic risk factors, BRCA2 mutations are associated with an increase of the risk of cancer of at least 3,5- and up to 10-fold, whereas a possible link with BRCA1 mutations has been observed. Other genetic risk factors include Peutz-Jeghers syndrome (STK11/LKB1 mutations) with a 132-fold risk, familial atypical multiple mole melanoma syndrome (CDKN2A mutations) with 13-22-fold increase of the risk, familial adenomatous polyposis (FAP) with a risk increased 4-fold, Lynch syndrome, with a risk increased 8.6-fold, and hereditary pancreatitis (PRSS1 mutations) with a 53-fold risk.

The Authors of the present invention have discovered that by analyzing the protein crown resulting from contacting gold nanoparticles with blood samples of patients suffering from adenocarcinoma it was possible to identify anomalies in the overall protein expression by low-cost techniques, such as, e.g., polyacrylamide gel electrophoresis (PAGE) of proteins by using sodium-dodecyl-sulfate (SDS). SDS-PAGE is a low-cost, fast experimental technique, widespread in clinical laboratories. The low resolution of electrophoresis, though not allowing to identify individual tumor biomarkers (as is done with mass spectrometry), enabled to assess "overall variations" in the protein profile even without a priori hypotheses on possible alterations in question, and in the detection of a profile of multiple proteins whose expression is modified overall in connection with the presence of the pathology.

In short, the present invention is based on the difference detected in the overall composition of the protein crown formed around gold nanoparticles of size equal to 100 nm after interaction with human plasma from healthy subjects and patients suffering from pancreatic adenocarcinoma. Crown overall composition was characterized by SDS-PAGE experiments. The study whose results are reported in the present patent application was financed by AIRC (Italian Association for Cancer Research) (IG 2017 ID. 20327; Principal Investigator: Prof. Giulio Caracciolo) and approved beforehand by the Ethics Committee of the *Università Campus Bio-Medico* of Rome (Protocol number: 10.3(12).18; Amendment 3 of 09/18/2018).

Compared to what described in Caputo et al 2017, the invention differs in: i) the use of gold nanoparticles in lieu of liposomes; ii) the gel image processing procedure (absent in the cited work); iii) the methodology of one-dimensional protein profile processing and input variable identification. The use of gold nanoparticles in lieu of liposomes is essential for the clinical application of the blood test. In fact, colloidal dispersions of gold nanoparticles are commercial, have a low cost, are ready for use and stable over time. Conversely, liposomes need a complex preparation procedure from powdered lipid species, required a dedicated instrumentation (balance, rotavapor, ultrasound extrusor/sonicator) and are stable over a short period of time if stored at 4°C.

No indication is provided, neither in the prior art nor in the cited document, for image processing, experimental data treatment and statistical techniques of experimental data treatment so as to obtain relevant results in the early diagnosis of pancreatic adenocarcinoma.

Therefore, the present invention relates to a method to assist in the early diagnosis of pancreatic adenocarcinoma which allows the identification of subjects in an early state of disease or at risk of pancreatic adenocarcinoma for whom it is necessary or appropriate to carry out the complex examinations conventionally used today (herein referred to as second-level) which normally are carried out in an often belated manner, no tests being available, to date, which allow a screening to identify in populations potentially at risk of disease, potentially diseased patients (in the present description also referred to as potentially pathological) and patients for whom the actual risk be validated by laboratory tests and not merely by the patient's medical and family history. As to date no diagnostic tests are available which allow examinations for the carrying out of an early screening of the risk or of the potential presence of the pancreatic tumor, the test subject matter of patent would therefore represent a first-level test, for a better identification of patients already potentially diseased, or in which the risk of disease be effectively validated by a laboratory examination. The examinations currently carried out, when the general pathological conditions of the patient indicate a presumable state of disease (and therefore normally when the disease is actually present and at an advanced stage) are defined herein as second-level examinations, and are represented by CT, MRI, ERCP and echoendoscopy. The method of the invention is based on a classifying of a plasma sample of these subjects according to two or more levels of risk in which the classification of said sample in at least one of said levels of risk indicates the need or opportunity to carry out second-level examinations, comprising the following steps:

a) providing a plasma sample from a blood sample of a subject to be analyzed;

b) incubating said plasma sample with gold nanoparticles so as to allow the formation of a protein crown on said nanoparticles, said passage b) being optionally followed by a passage b') in which the incubated material is subjected to centrifugation and one or more washes with saline phosphate buffer to eliminate weakly bound proteins;

c) separating the proteins that make up said protein crown from said nanoparticles;

d) subjecting said proteins to electrophoresis on a denaturing polyacrylamide gradient gel so as to obtain the protein profile (Pp) of the protein crown obtained in point b) or b');

e) providing at least one discriminant function ($f(\alpha_1, \alpha_2, \alpha_3, ..... \alpha_n)$, $g(\alpha_1, \alpha_2, \alpha_3, ..... \alpha_n)$) and two or more bands of molecular weight ($A_1, A_2...A_n$), said at least one discriminant function ($f(\alpha_1, \alpha_2, \alpha_3, ..... \alpha_n)$, $g(\alpha_1, \alpha_2, \alpha_3, ..... \alpha_n)$) and said bands of molecular weight ($A_1, A_2, A_3, ....A_n$) being predetermined on the basis of a set of reference samples; (said reference samples being represented by a population collected from healthy subjects and from subjects suffering from overt pancreatic tumor);

f) calculating an integral area value ($VA_1, VA_2... VA_n$) of said protein profile (Pp) for each of said bands of molecular weight ($A_1, A_2... A_n$);

g) calculating at least one discriminant value ($VD_f, VD_g$) of said at least one discriminant function ($f(\alpha_1, \alpha_2, \alpha_3, .... \alpha_n)$, $g(\alpha_1, \alpha_2, \alpha_3, .... \alpha_n)$) for at least one pair of said integral area values ($VA_1, VA_2... VA_n$);

h) classifying the plasma sample by comparing said at least one discriminant value ($VD_f, VD_g$) with at least one predefined threshold value (VS).

GLOSSARY

In the present description the term protein crown (corona) has the meaning commonly used in the scientific literature, and it denotes the layer of proteins adsorbed on the surface of the nanoparticles following interaction with biological fluids (e.g., blood and plasma).

For the purposes of the invention and of the present description, therefore, the first-level examination is represented by the examination carried out with the method of the present invention, whereas the second-level examinations are represented by those conventionally carried out to date, which are at least one among abdomen CT, abdomen MRI, echoendoscopy, ERCP (endoscopic retrograde cholangiopancreatography). Notably, said second-level examinations, being complex and costly for the patient or the national health system, are normally carried out when the patient's general health conditions show concrete symptoms of the disease, and are therefore normally carried out at a late stage of the disease, which, as indicated in the discussion of the state of the art, usually leads to the patient's death.

For the purposes of the present description, by protein profile it is meant the distribution of the molecular weights of the proteins that make up the protein crown.

DESCRIPTION OF THE FIGURES

Fig. 1 shows a schematic exemplary diagram of the method of the invention;
Fig. 2a is a representative image of a gel;
Fig. 2b is the result of a processing of Fig. 2a, after bottom subtraction;
Fig. 2c is a graph showing an intensity surface of a generic sample of the gel and a corresponding one-dimensional profile;
Fig. 2d is a graph showing the location of the reference bands through a nonlinear functional relationship;
Fig. 3a is a graph showing two one-dimensional profiles representative of the protein crown adsorbed on the surface of gold nanoparticles of size equal to 100 nm after incubation in the plasma of a healthy subject (profile 'o') and of a patient suffering from pancreatic adenocarcinoma (profile 'x');
Fig. 3b are graphs of the distributions of four possible integral areas showing significant differences between healthy and diseased subjects: 10-20 kDa; 20-25 kDa; 25-35 kDa; 35-45 kDa;
Fig. 3c reports two-dimensional graphs for each possible pair of multivariate distributions;
Fig. 4 is a two-dimensional graph referred to a selected pair of multivariate distributions; and
Fig. 5 is a three-dimensional graph referred to a selected triplet of multivariate distributions.

DETAILED DESCRIPTION

The present method to assist in the early diagnosis of pancreatic adenocarcinoma which allows the identification of subjects in an early state of disease or at risk of pancreatic adenocarcinoma for whom it is necessary or appropriate to carry out second-level examinations by classifying a plasma sample of these subjects according to two or more levels of risk in which the classification of said sample in at least one of said levels of risk indicates the need or opportunity to carry out second-level examinations, comprising the following steps:

a) providing a plasma sample from a blood sample of a subject to be analyzed;
b) incubating said plasma sample with gold nanoparticles so as to allow the formation of a protein crown on said nanoparticles, said passage b) being optionally followed by a passage b') in which the incubated material is subjected to centrifugation and one or more washes with phosphate buffer to eliminate weakly bound proteins;
c) separating the proteins that make up said protein crown from said nanoparticles;
d) subjecting said proteins to electrophoresis on a denaturing polyacrylamide gradient gel and processing the image of said gel so as to obtain the protein profile (Pp) of the protein crown obtained in point b) or b');
e) providing at least one discriminant function ($f(\alpha_1, \alpha_2, \alpha_3, ..... \alpha_n)$, $g(\alpha_1, \alpha_2, \alpha_3, ..... \alpha_n)$) and two or more bands of molecular weight ($A_1, A_2...A_n$), said at least one discriminant function ($f(\alpha_1, \alpha_2, \alpha_3, ..... \alpha_n)$, $g(\alpha_1, \alpha_2, \alpha_3, ..... \alpha_n)$) and said bands of molecular weight ($A_1, A_2, A_3, ....A_n$) being predetermined on the basis of a set of reference samples;
f) calculating an integral area value ($VA_1, VA_2... VA_n$) of said protein profile (Pp) for each of said bands of molecular weight ($A_1, A_2... A_n$);
g) calculating at least one discriminant value ($VD_f, VD_g$) of said at least one discriminant function ($f(\alpha_1, \alpha_2, \alpha_3, .... \alpha_n)$, $g(\alpha_1, \alpha_2, \alpha_3, .... \alpha_n)$) for at least one pair of said integral area values ($VA_1, VA_2... VA_n$);
h) classifying the plasma sample by comparing said at least one discriminant value ($VD_f, VD_g$) with at least one predefined threshold value (VS).

As indicated above, the set of reference samples is represented by samples coming from healthy individuals and samples of patients suffering from pancreatic adenocarcinoma. Said set of reference samples may be the set used and analyzed in the present description, or may be a set different from the one used in the present description, for which at least one discriminant function ($f(\alpha_1, \alpha_2, \alpha_3, .... \alpha_n)$, $g(\alpha_1, \alpha_2, \alpha_3, .... \alpha_n)$) and two or more bands of molecular weight ($A_1, A_2...A_n$) are predetermined as defined hereinafter. The method of the invention can in fact comprise a step (passage) of predetermining at least one discriminant function and at least two bands of molecular weight preceding step e, on the protein profiles of the reference samples obtained, them also, as described in points a) to d) of the method claimed and in the detailed description of the invention. The method of the invention therefore allows to subdivide the analyzed patients on the basis of two or more levels of risk, in which the two basic levels are not at risk and at risk, with a sensitivity, specificity and overall correctness each greater than 75%, each preferably greater than 77%, or, even more preferably, each greater than 78%. Furthermore, levels intermediate between the two above-indicated ones can be identified, such as, e.g. low-risk and high-risk.

A sample's belonging to one of these levels of risk is determined as described in greater detail below.

In particular, the sensitivity and the overall correctness can also be each greater than 80%, or even each greater than 81%.

In the embodiment of the invention reported also in the examples, the observed final values of sensitivity, specificity and overall correctness are: 83.7%, 78.6%, 81.8%, respectively.

For the purposes of the present invention, e.g. for the above-reported values, the sensitivity, specificity and overall correctness of the method of the invention can be calculated on the basis of the number of correctly classified elements, as follows:

- sensitivity=number of subjects classified as at risk or potentially diseased/number of actually diseased subjects.
- specificity=number of subjects classified as not at risk/number of actually healthy subjects.
- overall correctness=number of correctly classified subjects/total number of subjects.

According to the present invention, and as indicated in the glossary, the first-level examination, in connection with the diagnosis of pancreatic adenocarcinoma comprises, is represented by the examination carried out by the method of the invention.

For the purposes of the carrying out of the method of the invention, said second-level examinations comprise one or more among abdomen CT, abdomen MRI, echoendoscopy, ERCP.

According to the invention, in point b) gold nanoparticles having an average diameter of 100 nm can be used. Also gold nanoparticles having an average diameter of 100 nm $\pm$ 20 nm are useful for carrying out the invention.

The incubation can be carried out, for instance, by mixing two identical or alike volumes of serum and of nanoparticles in suspension as provided by the manufacturer, and by incubating the whole (mixture) for a defined time and temperature.

Serum incubation with the gold nanoparticles can be carried out at a temperature between 35 and 40°C, preferably between 36 and 39°C, even more preferably at about 37°C.

The incubation time is preferably between 40 and 120 minutes, like e.g., a period of time from 50 to 70 minutes, or also a period of about one hour.

In a specific embodiment, said incubation can be carried out at about 37°C for a period of time of about one hour.

In order to concentrate the nanoparticles, the serum can be subjected to centrifugation after the incubation period; the expert in the field will readily set the centrifugation times and rpms, which could be, in a merely indicative form, about 12-18 minutes at about 12000-15000 rpm. Preferably, the material previously incubated will be kept at temperatures of about 4°C.

Optionally, the proteins-nanoparticles complex, freed from the supernatant, could be subjected to one or more passages b') of washing with a suitable solution to eliminate weakly bound proteins. A suitable solution is represented, merely by way of example, by saline phosphate buffer.

The above-described process leads to the obtainment, after the last centrifugation (the washing passages will consist in suspending the pellet in an appropriate solution, centrifugation, supernatant removal, resuspension, etc.) from gold nanoparticles coated with the protein crown.

According to the method of the invention, the obtained pellets are resuspended by centrifuging the product of point b) or of point b') in a solution suitable to load the material suspended therein in a denaturing gel. In particular, the same solution normally is denaturing and contains, e.g., SDS. A passage of some minutes (8-13') at boiling temperature of the water in which test tubes are placed, containing the solution in which the product of point b) or of point b') is resuspended after centrifuging, leads to the detachment of the protein crown from the gold nanoparticles (point c) of the method), thereby allowing to isolate the protein crown and load it onto a polyacrylamide gradient gel in order to obtain a protein profile (point d) of the method).

The gel could, e.g., have a 4-20% polyacrylamide gradient.

The samples can be, e.g., suspended into 20 microliters of solution, and about 10 microliters/well can then be loaded onto the gel.

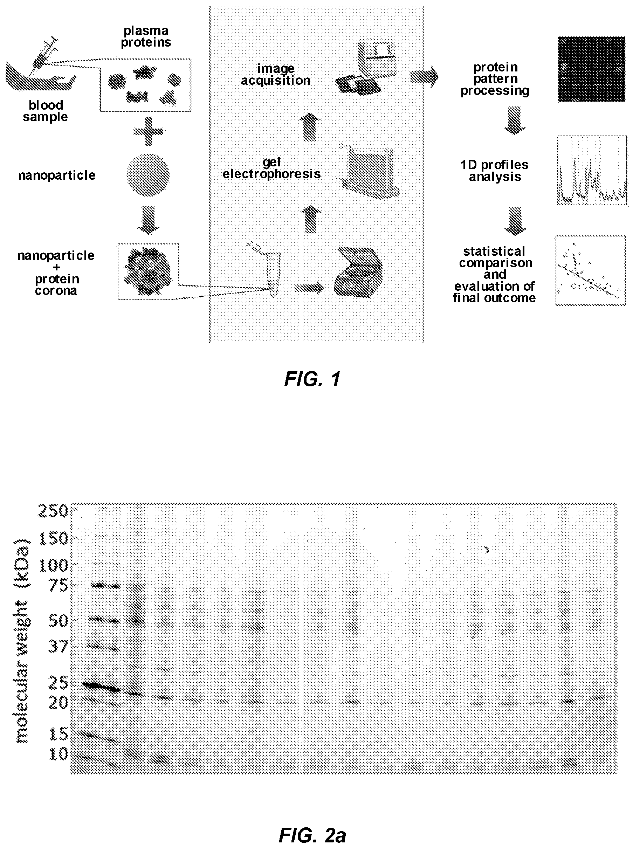

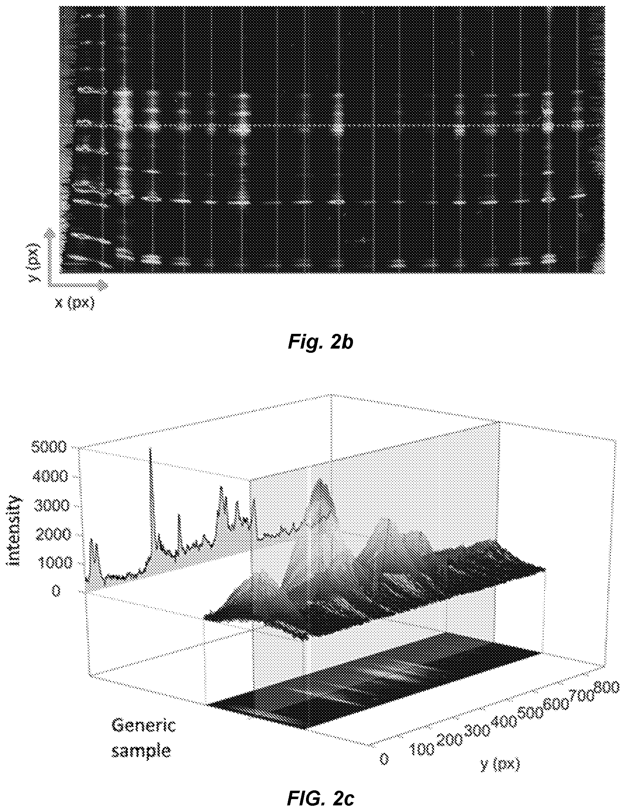

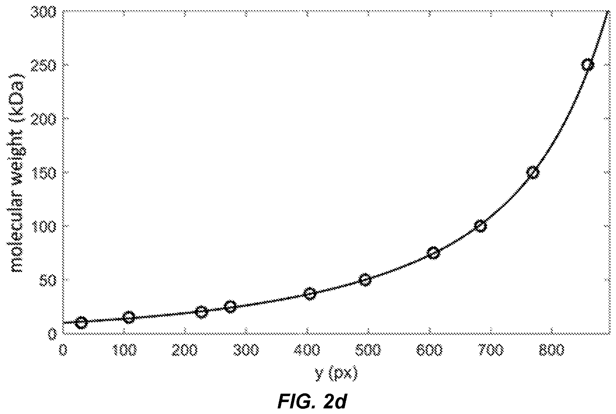

The gels containing the protein profile of the loaded samples are then subjected to image acquisition (e.g., by CCD camera, and appropriate image acquisition programs) and the image can then be processed, e.g. by bottom cleaning and subsequent processing by suitable codes in order to obtain a graphical representation, reported on abscissa and median, with intensity peaks, like that shown in Fig. 2.

For instance, the gels can be transferred inside an image acquisition system. By way of example, a Chemidoc MP system (Bio-Rad) can be used, containing a CCD (Charge-Coupled Device) camera to capture images, preferably high-quality ones, in real time, and allowing to accurately position the sample.

Gel images are processed to obtain a protein profile for each sample of the measurement.

A representative image of gel is shown in Fig. 2a, where the first lane represents a reference ladder for the spatial localization of the molecular weights and the remaining lanes correspond to the loaded samples.

Preliminarily, a removal of the bottom signal is preferably carried out to prevent systematic variations of exposure or luminosity from affecting the resulting profiles. According to one embodiment, for each image, the bottom subtraction technique via ball pivoting, row by row, is adopted. The ball radius is kept preferably constant for all processed images. Fig. 2b shows a representation of the image of a gel, after having subtracted the bottom. The left (left of the lane containing the reference ladder) and right (right of the last lane of the gel) sides show the presence of residual regions, inside which a marked pattern can be recognized, resembling that of the intensity of the original image.

Formally, if $I(x, y)$ represents the intensity sampled from the original image of a gel, the sampled image $(x, y)$ is obtained after the bottom removal procedure:

$$I(x, y) \rightarrow I'(x, y) \tag{1}$$

In other words, each image so processed can be considered as a function $I'$ of two spatial variables ($x$ and $y$), in which the shift $y$ is related to the molecular weights of the proteins (see Eq. 3) and the intensity value is related to the amount of proteins. FIG. 2c shows a spatial representation of function $I'$.

A projection $P$ of the two-variable function $I'(x, y)$ on a plane orthogonal to the image represents the resulting protein profile for a generic sample ($j$):

$$P_j(y) = I'(x_j, y) \tag{2}$$

Finally, the shifts $y$ can be converted into molecular weights by comparing the position of the known proteins (first lane) with a nonlinear equation of the type

$$f(y) = a1^* e^{(b1^*y)} + a2^* e^{(b2^*y)} \tag{3}$$

Where parameters a1, b1, a2, b2 are obtained via an experimental data fit procedure (position of bands corresponding to known proteins) by the functional relationship f. For any fit procedure the determination coefficient $R^2$ can be calculated as follows:

$$R^2 = 1 - S_r/S_t$$

Where $S_r$ is the sum of the squares of the residues (each residue is defined as the difference between the experimental data and the value assumed by the fit function at the experimental data itself) and $S_t$ is the sum of the squares of the differences between the experimental data and the mean of the experimental data.

Among the infinite possible functional relationships, the two-term exponential curve formalized in equation (3) represents a very reasonable, and above all effective choice, as it allows a very stable and versatile fit procedure. With this choice the determination parameter, $R^2$, expressing the good quality of the procedure itself, settles on values not lower than 0.9999 (where 1 is the maximum reachable). Otherwise, $R^2$ is about 0.9894 for single-term exponential curves, 0.9929 and 0.9499 for third- and second-degree polynomials, 0.9614 and 0.9915 for single- and double-term power laws.

It is understood that these values of the determination parameter $R^2$ are related to the reference data set considered herein by way of example. If another data set were used, as described herein, e.g., adding new samples, or starting from a different set of samples, $R^2$ values might vary.

Thus, at the end of the image processing procedure, for each sample a one-dimensional protein profile is obtained which expresses the distribution of the molecular weight of the proteins that make up the crown. There are commercial and free programs to process gel images, like, e.g., programs such as ImageJ (Mathworks) and Image Lab™ (Bio-Rad) or the like. Preferably, the programs could be designed or modified to allow a bottom subtraction, so as to guarantee reproducibility in comparing different gels.

The protein profiles obtained from gel electrophoresis experiments for a set of known reference samples, with the same modes described above with regard to the samples to be analyzed, are input in an appropriate database, so as to associate each sample (healthy or diseased) with its personal profile.

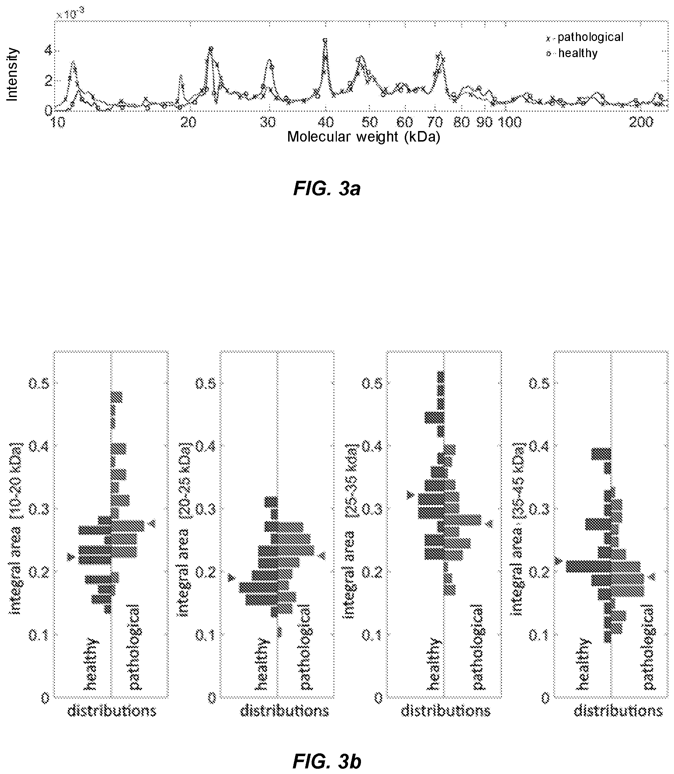

By way of example, Fig. 3a shows two one-dimensional profiles representative of the protein crown adsorbed on the surface of gold nanoparticles of size equal to 100 nm after incubation in the plasma of a healthy subject (marked with 'o') and of a patient suffering from pancreatic adenocarcinoma (marked with 'x').

Sample classification and the entailed distinction between subjects not at risk and patients at risk or potentially diseased is, according to the invention, carried out through statistical analyses.

In particular, on the basis of the set of reference samples, the overall molecular weight interval is subdivided into two or more bands of molecular weight $A_1, A_2... A_n$ (n being an integer greater than 2) so that each peak - corresponding to a respective electrophoretic band - of each protein profile of the set of reference samples belongs to one and only one of said bands. Said bands are selected so as not to cut the peaks observable in the overall protein profile. For protein band determination, the profiles of the reference set are considered individually and the determination of the bands of molecular weight is univocal for the entire set of data under consideration.

In one embodiment of the invention said bands can be, for example, two or more among $A_1$: 10-20 kDa; $A_2$: 20-25 kDa; $A_3$: 25-35 kDa; $A_4$: 35-45 kDa.

Fig. 3b shows four corresponding distributions of integral area values calculated for each of the four above-indicated bands of molecular weight.

The integral area values are used as input parameters for the subsequent computation, i.e. for a phase of classification of the subjects according to two or more levels of risk as defined herein. Said classification phase is, according to the invention, carried out through a linear discrimination analysis.

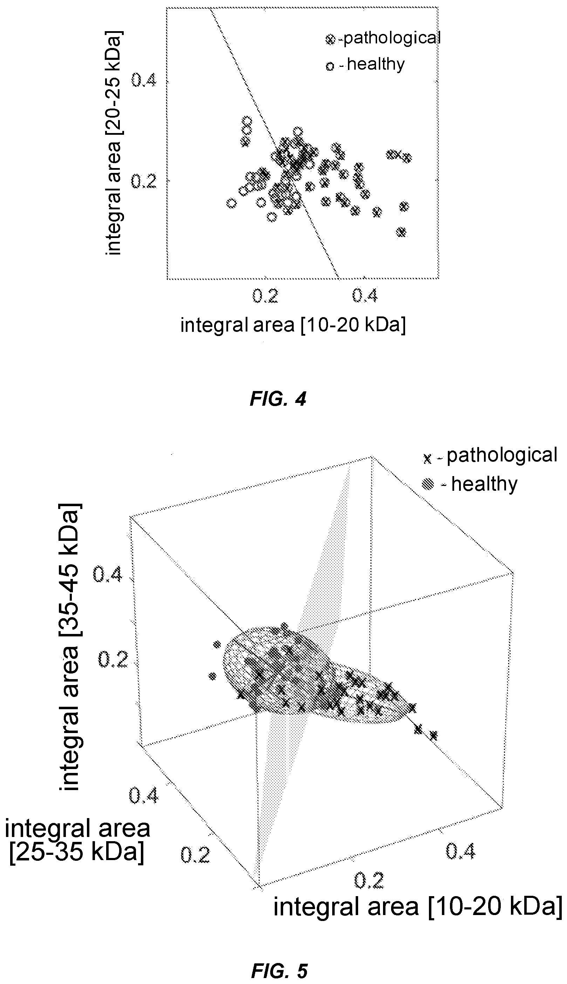

Said linear classification approach can be applied both in two dimensions (Fig. 4) and in three dimensions (Fig. 5), corresponding respectively to pairs and triplets of multivariate distributions.

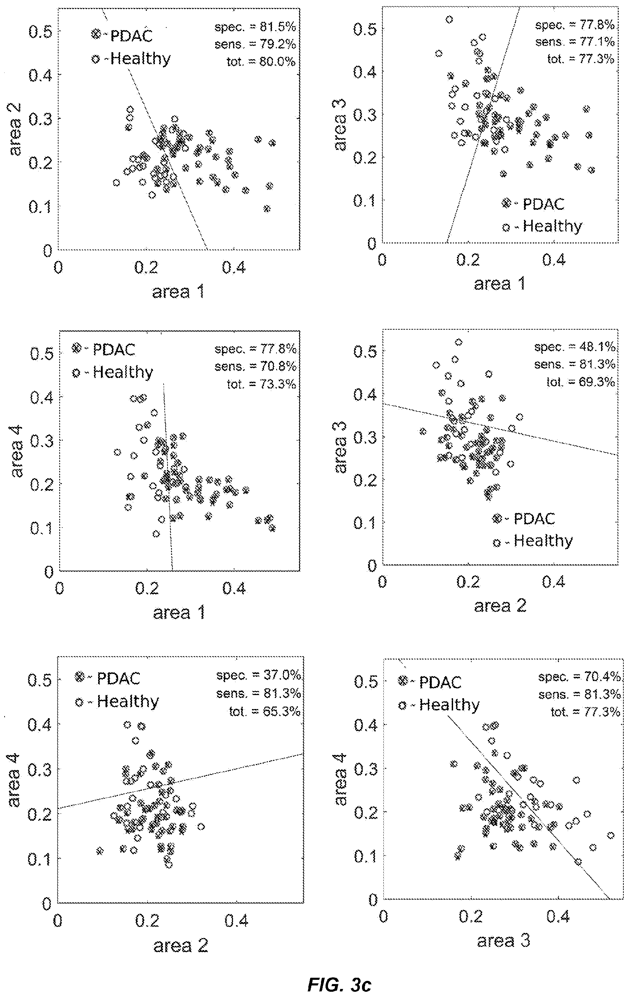

Figure 3c shows the distribution of the integral area values, for all possible pairs of multivariate distributions.

The best pair is that maximizing the overall correctness of the test as described hereinafter.

Linear discrimination analysis is a statistical methodology well-known in the art, formalized by R. A. Fisher in 1936, and represents one of the first and major references for the development of additional procedures underlying modern machine learning and data mining. Hence, it is not deemed necessary to delve into the details of said methodological approach, it being certainly within the reach of an expert in the field.

According to the present invention at least one linear discriminant function $f(\alpha_1, \alpha_2)$, $g(\alpha_1, \alpha_3, \alpha_4)$ was found maximizing the ratio of the differences between the specific single-class mean values, compared to standard deviations of the experimental variables. The result can be displayed graphically both as a straight line (in two dimensions, Fig. 4) or as a plane (in three dimensions, Fig. 5), subdividing the parameter space (the areas of the electrophoretic profiles) into two regions: that associated with the distribution of healthy samples and that corresponding to the counterpart of the pathological subjects or of subjects at risk, probably suffering from pancreatic tumor. The generic unknown sample will therefore be classified based on its position in the parameter space of the areas.

According to a preferred embodiment of the invention, the discriminant function comprises a first function $f(\alpha_1, \alpha_2)$ represented by the equation of a straight line of the type:

$$f(\alpha_1, \alpha_2) = r1 + r2 * \alpha_1 + r3 * \alpha_2 = 0$$

in which r1, r2 and r3 are the coefficients of the line and $\alpha_1$ and $\alpha_2$ are integral area values.

According to one embodiment, and on the basis of the set of reference samples used as described in the examples that follow, the coefficients of the line assume the following values:

r1 = - 7.513
r2 = + 22.120
r3 = + 9.659

Function $f(\alpha_1, \alpha_2)$ will therefore assume the following form:

$$f(\alpha_1, \alpha_2) = -7.513 + 22.120\alpha_1 + 9.659\alpha_2 = 0$$

According to the example described herein, $\alpha_1$ represents the (integral area value) value of integral area defined by the band of molecular weight $A_1$ between 10 and 20 kDa, and $\alpha_2$ the integral area value in band $A_2$ between 20 and 25 kDa.

According to the present invention, at least one predefined threshold value VS is set, with which to compare the discriminant value $VD_f$ of the discriminant function $f(\alpha_1, \alpha_2)$, calculated for the pair of integral area values $\alpha_1$ and $\alpha_2$ as defined above.

From comparison, the unknown plasma sample is classified as potentially pathological (also referred to as potentially diseased) or at risk if the discriminant value VDf is greater than zero, otherwise it is classified as patient not at risk. Of course, as already indicated, more than two levels of potentially pathological or at risk of developing the pathology can be envisaged, therefore carrying out a classification in more than two classes, simply by setting more than one threshold value.

Moreover, given its simplicity and non-invasiveness, the examination carried out with the method of the invention can be repeated at successive times on previously examined samples in order to confirm obtained results or identify patients previously not at risk, who have, over time, undergone modifications of the protein crown that may move them into the at risk or the potentially pathological class, on which it is therefore strongly advisable to carry out the second-level examinations as defined herein.

Finally, the sensitivity, specificity and overall correctness of the test were calculated on the basis of the number of correctly classified elements, as follows:

- sensitivity=number of subjects classified as diseased/number of actually diseased subjects.
- specificity=number of subjects classified as healthy/number of actually healthy subjects.
- overall correctness=number of correctly classified subjects/total number of subjects.

Analogously, the at least one discriminant function may also comprise a second function $g(\alpha_1, \alpha_3, \alpha_4)$ represented by the equation of a plane of the type:

$$g(\alpha_1, \alpha_3, \alpha_4) = p1 + p2 * \alpha_1 + p3 * \alpha_3 + p4 * \alpha_4 = 0$$

in which p1, p2, p3 and p4 are coefficients of the plane and $\alpha_1$, $\alpha_3$ and $\alpha_4$ are integral area values.

According to one embodiment, and on the basis of the set of reference samples used as described in the examples that follow, the coefficients of the plane can assume the following values:

p1 = 0.139
p2 = + 2.301
p3 = - 1.517
p4 = 1.

Function $g(\alpha_1, \alpha_3, \alpha_4)$ will therefore assume the following form:

$$g(\alpha_1, \alpha_3, \alpha_4) = 0.139 + 2.301\alpha_1 - 1.517\alpha_3 - \alpha_4 = 0$$

According to the example described herein, $\alpha_1$ represents the (integral area value) value of the integral area defined by the band of molecular weight $A_1$ between 10 and 20 kDa, $\alpha_3$ the integral area value in band $A_3$ between 25 and 35 kDa, and $\alpha_4$ the integral area value in band $A_4$ between 35 and 45 kDa.

According to the present invention, at least one predefined threshold value VS is set, with which to compare the discriminant value $VD_g$ of the discriminant function $g(\alpha_1, \alpha_3, \alpha_4)$, calculated for the triplet of integral area values $\alpha_1$, $\alpha_3$ and $\alpha_4$ as defined above. From comparison, the unknown plasma sample is classified as pathological or at risk if the discriminant value $VD_g$ is greater than zero, otherwise it is classified as not at risk. Of course, as already indicated, more than two levels of pathological or at risk can also be envisaged, therefore carrying out a classification in more than two classes, simply by setting more than one threshold value.

As indicated above, having established that a sample falls within the "at risk" classification (VDg and/or VDf greater than 0) additional intermediate levels of risk can be defined. A possible example, according to the present invention, is represented by the levels defined as 'low-risk' or 'high-risk'. These additional levels can be identified in specific values of VDf and/or VDg; for instance: when the value of VDg and/or VDf is greater than 0 and ≤0.25 the sample can be further classified as " low-risk", therefore with a risk lower than samples classified as "high-risk". When the value of VDg and/or VDf is instead greater than 0.25 the sample can be classified as high-risk.

Not wishing to use the low- or high-risk definitions, the levels defined herein can also be defined as

Level 0 (not at risk)
Level 1 (at risk)
Level 2 (low risk, i.e., with a risk level lower than level 3)
Level 3 (high risk, i.e., with a risk level higher than level 2)

Finally, the sensitivity, specificity and overall correctness of the test were calculated on the basis of the number of correctly classified elements, as follows:

- sensitivity=number of subjects classified as at risk/number of actually diseased subjects.
- specificity=number of subjects classified as not at risk/number of actually healthy subjects.
- overall correctness=number of correctly classified subjects/total number of subjects.

Through the proposed method, the following final values of sensitivity, specificity and overall correctness were obtained: 83.7%, 78.6%, 81.8%, respectively.

In the example described herein, a set of 77 known reference samples (28 samples from healthy volunteers and 49 from oncological patients suffering from pancreatic adenocarcinoma) was used, and data analysis on the indicated pair $(\alpha_1, \alpha_2)$ returned the maximum value of overall correctness of the test (80%) among all possible pairings, therefore the abovementioned areas represent the best combination in a two-dimensional space.

Moreover, the classification carried out by considering a triplet of values returned the best values. By way of example, with reference to the examples reported below, it was observed that the overall correctness of the test improves of about 1.8% in passing from two to three dimensions.

EXAMPLES

## 1. Plasma production and storage

Plasma taken and collected in test tubes containing K2EDTA protease inhibitors (test tubes TM BD P100 Blood Collection System (Franklin Lakes, NJ, 7 USA) is subjected to centrifugation twice for 10 minutes at 2500 rpm for human plasma isolation (Fig. 1). Obtained plasma can be stored in Eppendorf (tubes) at -80°C until use. Before use, the human plasma is thawed at 4 °C.

## 2. Protein crown formation, isolation and characterization

Subsequently, a volume of 50 microliters of gold nanoparticles (Sigma Aldrich, Milan, Italy) is incubated with 50 microliters of human plasma for 1 hour at 37 °C. After incubation, samples are centrifuged at 14,000 rpm for 15 min at 4°C and subsequently washed thrice with saline phosphate buffer to eliminate weakly bound proteins. This step of the procedure causes precipitation of a pellet made up of coated gold nanoparticles of the protein crown. Pellets thus obtained are resuspended into 20 microliters of 1x loading solution (made up of: 50 mM Tris-HCl, 0.01% bromophenol blue (BPB), 0.5% sodium-dodecyl-sulfate (SDS), 10% glycerol, 0.1 M dithiothreitol (DTT)) and subsequently boiled for 10 minutes at 100°C. This step allows to isolate the protein crown from the gold nanoparticles. Finally, for each sample of the measurement 10 microliters are loaded on a polyacrylamide gradient gel (4-20%) at the ends of which a difference of potential equal to 100 mV is applied for about 90 minutes. Gels are then washed in bidistilled water.

## 3. Image acquisition and processing

Gels are transferred inside an image acquisition system Chemidoc MP (Bio-Rad) containing a CCD (Charge-Coupled Device) camera to capture images in real time and allow to accurately position the sample, thus generating optimized image data. Gel images are processed through Matlab codes (Mathworks), written by the Inventors and expressly validated for electrophoresis data processing, in order to obtain a protein profile for each sample of measurement. A representative gel image is shown in Fig. 2a, where the first lane represents a reference ladder for the spatial localization of the molecular weights and the remaining lanes correspond to the loaded samples. Preliminarily, a removal of the bottom signal is carried out to prevent systematic variations of exposure or luminosity from affecting the resulting profiles. In detail, for each image, the bottom subtraction technique via ball pivoting, row by row, is adopted. Ball radius was kept constant for all processed images. Fig. 2b shows a pseudocolor representation of the image of a gel, after having subtracted the bottom. The left (left of the lane containing the reference ladder) and right (right of the last lane of the gel) sides show the presence of residual regions, inside which a marked pattern can be recognized, resembling that of the intensity of the original image.

Formally, if I (x, y) represents the intensity sampled from the original image of a gel, the sampled image (x, y) is obtained after the bottom removal procedure:

$$I\,(x,\,y) \to I\,'(x,y) \qquad\qquad\qquad (1)$$

In other words, each image can be considered as a function (I') of two spatial variables (x and y), in which the shift y is related to the molecular weights of the proteins and the intensity value is related to the amount of proteins (see Figure 2).

The projection (P) of this two-variable function on a plane orthogonal to the image is the resulting protein profile for the generic sample (j) (Fig. 2c):

$$P_j\ (y) = I'(x_j,\ y) \tag{2}$$

Finally, the shifts y can be converted into molecular weights by comparing the position of the known proteins (first lane) with a nonlinear equation (Fig. 2d) of the type

$$f\ (y) = a1{*}e^{(b1{*}y)} + a2{*}e^{(b2{*}y)} \tag{3}$$

Among the infinite possible functional relationships, the two-term exponential curve formalized in Eq. 3 represents a very reasonable, and above all effective choice, as it allows a very stable and versatile fit procedure. For that matter, the determination parameter R2, expressing the good quality of the procedure itself, settles on values not lower than 0.9999 (1 is the maximum reachable) using Eq. 3, but is about 0.9894 for single-term exponential curves, 0.9929 and 0.9499 for third- and second-degree polynomials, 0.9614 and 0.9915 for single- and double-term power laws. Anyhow, at the end of the image processing procedure, for each sample a one-dimensional protein profile (Fig. 2c) is obtained which expresses the distribution of the molecular weight of the proteins that make up the crown.

### 4. Statistical processing of one-dimensional protein profiles and blood test predictive ability

The protein profiles obtained from gel electrophoresis experiments were input in an appropriate database, so that each subject (healthy or diseased) was associated with its personal adsorbed. Fig. 3a shows two one-dimensional profiles representative of the protein crown adsorbed on the surface of gold nanoparticles of size equal to 100 nm after incubation in the plasma of a healthy subject (blue profile) and of a patient suffering from pancreatic adenocarcinoma (orange profile).

Sample classification and the entailed distinction between healthy subjects and oncological patients is carried out through statistical analyses. In particular, each profile is subdivided into bands of molecular weight, so that each electrophoretic band belongs to one and only one of said intervals.

Fig. 3b shows the distributions of the four integral areas showing significant differences between healthy and diseased subjects: 10-20 kDa; 20-25 kDa; 25-35 kDa; 35-45 kDa. The integral area values are used as input parameters for the subsequent computation: the classification healthy subjects/diseased subjects through a linear discrimination analysis. Said linear classification approach was applied in two dimensions (Fig. 3c) and in three dimensions (Fig. 3d), respectively corresponding to paired multivariate distributions and to triplets of multivariate distributions. Linear discrimination analysis is one of the most effective methods to determine whether significant differences between different classes do exist, and to identify the discriminant power of each variable. The approach was formalized by R. A. Fisher in 1936 and represents one of the first and major references for the development of further procedures at the basis of modern machine learning and data mining.

The linear function maximizing the ratio of the differences between the specific single-class mean values, compared to standard deviations of the experimental variables, was found. The methodology is well-known and the result can be displayed graphically as a straight line (in two dimensions) or a plane (in three dimensions) subdividing the parameter space (the areas of the electrophoretic profiles) into two regions: that associated with the distribution of non-oncological samples and that corresponding to the counterpart of the subjects suffering from pancreatic tumor. The generic unknown sample is therefore classified based on its position in the parameter space of the areas. In the proposed case, the equation of the linear discrimination (straight) line (Fig. 3c) is the following

$$f(\alpha_1, \alpha_2) = -7.513 + 22.120\alpha_1 + 9.659\alpha_2 = 0 \tag{4}$$

Where $\alpha_1$ represents the integral area defined between 10 and 20 kDa, and $\alpha_2$ the integral area in the 20-25 kDa range. A generic sample is therefore classified at risk, i.e., potentially diseased (potentially pathological) if the values of the areas of its electrophoretic profile meet the inequality f>0 in which the plasma sample is classified as potentially pathological (also referred to as potentially diseased) or at risk if the discriminant value VDf is greater than zero, otherwise it is classified as subject not at risk. Finally, sensitivity, specificity and overall correctness of the test were calculated on the basis of the number of correctly classified elements, as follows:

sensitivity=number of subjects classified as potentially diseased/number of actually diseased subjects.
specificity=number of subjects classified as not at risk/number of actually healthy subjects.
overall correctness=number of correctly classified subjects/total number of subjects.

77 samples were used (28 samples from healthy volunteers and 49 from oncological patients suffering from pancreatic adenocarcinoma) and data analysis on the indicated pair ($\alpha 1$, $\alpha 2$) returned the maximum value of overall correctness of the test (80%) among all possible pairings, therefore the above-mentioned areas represent the best combination in a bidimensional space. Moreover, the extension to the three-dimensional case is analogous and returned better results. In particular, the equation of the plane as discriminant linear geometric entity is the following:

$$g(\alpha_1, \alpha_3, \alpha_4) = 0.139 + 2.301\alpha_1 - 1.517\alpha_3 - \alpha_4 = 0 \qquad (5)$$

Where $\alpha_1$ represents the integral area defined between 10 and 20 kDa, $\alpha_3$ the integral area in the 25-35 kDa range, and $\alpha_4$ the integral area in the 35-45 kDa range. The generic sample is therefore classified as at risk (potentially diseased) if g>0, otherwise it is considered not at risk. Through the proposed method, the following final values of sensitivity, specificity and overall correctness were obtained: 83.7%, 78.6%, 81.8%, respectively. Any unknown samples can then be classified through assessment of the electrophoretic profile, measurement of the specified integral areas and comparison with Eq. 5.

## Claims

1. Method to assist in the early diagnosis of pancreatic adenocarcinoma which allows the identification of subjects in an early state of disease or at risk of pancreatic adenocarcinoma for whom it is necessary or appropriate to carry out second-level examinations by classifying a plasma sample of these subjects according to two or more levels of risk in which the classification of said sample in at least one of said levels of risk indicates the need or opportunity to carry out second-level examinations, comprising the following steps:

   a) providing a plasma sample from a blood sample of a subject to be analyzed;
   b) incubating said plasma sample with gold nanoparticles so as to allow the formation of a protein crown on said nanoparticles, said passage b) being optionally followed by a passage b') in which the incubated material is subjected to centrifugation and one or more washes with phosphate buffer to eliminate weakly bound proteins;
   c) separating the proteins that make up said protein crown from said nanoparticles;
   d) subjecting said proteins to electrophoresis on a denaturing polyacrylamide gradient gel so as to obtain the protein profile (Pp) of the protein crown obtained in point b) or b');
   e) providing at least one discriminant function ($f(\alpha_1, \alpha_2,... \alpha_n)$, $g(\alpha_1, \alpha_2, \alpha_3, ... \alpha_n)$) and two or more bands of molecular weight ($A_1, A_2...A_n$), said at least one discriminant function ($f(\alpha_1, \alpha_2,... \alpha_n)$, $g(\alpha_1, \alpha_2, \alpha_3, ... \alpha_n)$) and said bands of molecular weight ($A_1, A_2, .... A_n$) being predetermined on the basis of a set of reference samples;
   f) calculating an integral area value ($VA_1, VA_2... VA_n$) of said protein profile (Pp) for each of said bands of molecular weight ($A_1, A_2... A_n$);
   g) calculating at least one discriminant value ($VD_f, VD_g$) of said at least one discriminant function ($f(\alpha_1, \alpha_2, ... \alpha_n)$, $g(\alpha_1, \alpha_2, \alpha_3, ... \alpha_n)$) for at least one pair of said integral area values ($VA_1, VA_2... VA_n$);
   h) classifying the plasma sample by comparing said at least one discriminant value ($VD_f, VD_g$) with at least one predefined threshold value (VS).

2. Method according to claim 1, wherein said second-level examinations comprise one or more among: abdomen CT, abdomen MRI, echoendoscopy, ERCP.

3. Method according to claims 1 or 2, wherein said at least one discriminant function ($f(\alpha_1, \alpha_2,... \alpha_n)$, $g(\alpha_1, \alpha_2, \alpha_3, ... \alpha_n)$) is obtained by linear discrimination analysis.

4. Method according to any one of the preceding claims, wherein said at least one discriminant function ($f(\alpha_1, \alpha_2, ... \alpha_n)$, $g(\alpha_1, \alpha_2, \alpha_3, ... \alpha_n)$) comprises a first function ($f(\alpha_1, \alpha_2)$) represented by the equation of a straight line of the type:

$$f(\alpha_1, \alpha_2) = r1 + r2 * \alpha_1 + r3 * \alpha_2 = 0$$

   in which r1, r2 and r3 are the coefficients of the line and $\alpha_1$ and $\alpha_2$ are integral area values.

5. Method any one of claims 1 to 4, wherein said at least one discriminant function ($f(\alpha_1, \alpha_2, ... \alpha_n)$, $g(\alpha_1, \alpha_2, \alpha_3, ... \alpha_n)$) comprises a second function ($g(\alpha_1, \alpha_3, \alpha_4)$) represented by the equation of a plane of the type:

$$g(\alpha_1, \alpha_3, \alpha_4) = p1 + p2 * \alpha_1 + p3 * \alpha_3 + p4 * \alpha_4 = 0$$

in which p1, p2, p3 and p4 are coefficients of the plane and $\alpha_1$, $\alpha_3$ and $\alpha_4$ are integral area values.

6. Method according to any one of the preceding claims, wherein said two or more bands of molecular weight ($A_1$, $A_2$... $A_n$) are determined so that each peak - corresponding to a respective electrophoretic band - of each protein profile of the set of reference samples belongs to one and only one of said bands.

7. Method according to any one of claims 1 to 6, wherein said at least one predefined threshold value (VS) is equal to zero and said plasma sample is classified as at risk if the discriminant value ($VD_f$, $VD_g$) is greater than zero.

8. Method according to any one of claims 1 to 7, wherein said bands of molecular weight can be, for example, two or more among $A_1$: 10-20 kDa; $A_2$: 20-25 kDa; $A_3$: 25-35 kDa; $A_4$: 35-45 kDa.

9. Method according to one of claims 1 to 7, said gold nanoparticles have an average diameter of 100 nm.

10. Method according to any one of claims 1 to 9, step b) is carried out at a temperature between 35 and 40°C for a period of time between 40 and 120 minutes.

11. Method according to claim 10, wherein said temperature is 37°C and/or said time is 55 to 56 minutes.

12. Method according to any one of claims 1 to 11, wherein said step b) is followed by a step b') in which the incubated material is subjected to centrifugation and one or more washes with phosphate buffer to eliminate weakly bound proteins.

13. Method according to any one of claims 1 to 12, wherein said separation at point c) is carried out by boiling for about 10 minutes the gold particles as obtained in point b) or b') suspended in polyacrylamide gel charge buffer comprising SDS.

14. Method according to any one of claims 1 to 13, wherein said polyacrylamide gradient is a 4-20% gradient.

**Patentansprüche**

1. Verfahren zum Unterstützen bei der Frühdiagnose von Pankreasadenokarzinomen, das die Identifizierung von Subjekten ermöglicht, die sich in einem frühen Krankheitsstadium befinden oder für Pankreasadenokarzinome gefährdet sind, für die es notwendig oder angebracht ist, Untersuchungen einer zweiten Ebene durch Klassifizieren einer Plasmaprobe dieser Subjekte gemäß zwei oder mehreren Risikostufen durchzuführen, bei denen die Klassifizierung der Probe in mindestens eine der Risikostufen die Notwendigkeit oder Gelegenheit anzeigt, Untersuchungen der zweiten Ebene durchzuführen, umfassend die folgenden Schritte:

a) Bereitstellen einer Plasmaprobe aus einer Blutprobe eines zu analysierenden Subjekts;
b) Inkubieren der Plasmaprobe mit Goldnanopartikeln, um die Bildung einer Proteinkrone auf den Nanopartikeln zu ermöglichen, wobei auf die Passage b) optional eine Passage b') folgt, in der das inkubierte Material einer Zentrifugation und einem oder mehreren Waschvorgängen mit Phosphatpuffer unterzogen wird, um schwach gebundene Proteine zu entfernen;
c) Trennen der Proteine, aus denen die Proteinkrone besteht, von den Nanopartikeln;
d) Unterziehen der Proteine einer Elektrophorese auf einem denaturierenden Polyacrylamidgradientengel, um das Proteinprofil (Pp) der Proteinkrone zu erhalten, die in Punkt b) oder b') erhalten wird;
e) Bereitstellen mindestens einer Diskriminanzfunktion ($f(\alpha_1, \alpha_2, ... \alpha_n)$, $g(\alpha_1, \alpha_2, \alpha_3, ... \alpha_n)$) und zweier oder mehrer Molekulargewichtsbänder ($A_1$, $A_2$...$A_n$), wobei mindestens eine Diskriminanzfunktion ($f(\alpha_1, \alpha_2, ... \alpha_n)$, $g(\alpha_1, \alpha_2, \alpha_3, ... \alpha_n)$) und die Molekulargewichtsbänder ($A_1$, $A_2$, .... $A_n$) auf der Grundlage eines Satzes von Referenzproben zuvor bestimmt wird;
f) Berechnen eines integralen Flächenwerts ($VA_1$, $VA_2$... $VA_n$) des Proteinprofils (Pp) für jedes der Molekulargewichtsbänder ($A_1$, $A_2$... $A_n$);
g) Berechnen mindestens eines Diskriminanzwerts ($VD_f$, $VD_g$) der mindestens einen Diskriminanzfunktion ($f(\alpha_1, \alpha_2, ... \alpha_n)$, $g(\alpha_1, \alpha_2, \alpha_3, ... \alpha_n)$) für mindestens ein Paar der integralen Flächenwerte ($VA_1$, $VA_2$... $VA_n$);
h) Klassifizieren der Plasmaprobe durch Vergleichen des mindestens einen Diskriminanzwerts ($VD_f$, $VD_g$) mit mindestens einem vordefinierten Schwellenwert (VS).

**2.** Verfahren nach Anspruch 1, wobei die Untersuchungen der zweiten Ebene eine oder mehrere umfassen von: CT eines Abdomens, MRI des Abdomens, Echoendoskopie, ERCP.

**3.** Verfahren nach Anspruch 1 oder 2, wobei die mindestens eine Diskriminanzfunktion ($f(\alpha_1, \alpha_2, ... \alpha_n)$, $g(\alpha_1, \alpha_2, \alpha_3, ... \alpha_n)$) durch lineare Diskriminierungsanalyse erhalten wird.

**4.** Verfahren nach einem der vorstehenden Ansprüche, wobei die mindestens eine Diskriminanzfunktion ($f(\alpha_1, \alpha_2, ... \alpha_n)$, $g(\alpha_1, \alpha_2, \alpha_3, ... \alpha_n)$) eine erste Funktion ($f(\alpha_1, \alpha_2)$) umfasst, dargestellt durch die Gleichung einer geraden Linie der Art:

$$f(\alpha_1, \alpha_2) = r1 + r2 * \alpha_1 + r3 * \alpha_2 = 0$$

wobei r1, r2 und r3 die Koeffizienten der Linie und $\alpha_1$ und $\alpha_2$ integrale Flächenwerte sind.

**5.** Verfahren nach einem der Ansprüche 1 bis 4, wobei die mindestens eine Diskriminanzfunktion ($f(\alpha_1, \alpha_2, ...\alpha_n)$, $g(\alpha_1, \alpha_2, \alpha_3, ... \alpha_n)$) eine zweite Funktion ($g(\alpha_1, \alpha_3, \alpha_4)$) umfasst, dargestellt durch die Gleichung einer Ebene der Art:

$$g(\alpha_1, \alpha_3, \alpha_4) = p1 + p2 * \alpha_1 + p3 * \alpha_3 + p4 * \alpha_4 = 0$$

wobei p1, p2, p3 und p4 Koeffizienten der Ebene sind und $\alpha_1$, $\alpha_3$ und $\alpha_4$ integrale Flächenwerte sind.

**6.** Verfahren nach einem der vorstehenden Ansprüche, wobei die zwei oder mehr Molekulargewichtsbänder ($A_1$, $A_2$... $A_n$) bestimmt werden, sodass jeder Peak - der einem jeweiligen elektrophoretischen Band entspricht - jedes Proteinprofils des Satzes von Referenzproben zu einem und nur einem der Bänder gehört.

**7.** Verfahren nach einem der Ansprüche 1 bis 6, wobei der mindestens eine vordefinierte Schwellenwert (VS) gleich Null ist und die Plasmaprobe als gefährdet eingestuft wird, falls der Diskriminanzwert ($VD_f$, $VD_g$) größer als Null ist.

**8.** Verfahren nach einem der Ansprüche 1 bis 7, wobei die Molekulargewichtsbänder, beispielsweise, zwei oder mehr sein können unter $A_1$: 10-20 kDa; $A_2$: 20-25 kDa; $A_3$: 25-35 kDa; $A_4$: 35-45 kDa.

**9.** Verfahren nach einem der Ansprüche 1 bis 7, wobei die Goldnanopartikel einen durchschnittlichen Durchmesser von 100 nm aufweisen.

**10.** Verfahren nach einem der Ansprüche 1 bis 9, wobei Schritt b) bei einer Temperatur zwischen 35 und 40 °C für einen Zeitraum zwischen 40 und 120 Minuten durchgeführt wird.

**11.** Verfahren nach Anspruch 10, wobei die Temperatur 37 °C und/oder die Zeit 55 bis 56 Minuten beträgt.

**12.** Verfahren nach einem der Ansprüche 1 bis 11, wobei auf den Schritt b) ein Schritt b') folgt, in dem das inkubierte Material der Zentrifugation und einem oder mehreren Waschvorgängen mit Phosphatpuffer unterzogen wird, um schwach gebundene Proteine zu entfernen.

**13.** Verfahren nach einem der Ansprüche 1 bis 12, wobei die Trennung in Punkt c) durch Kochen für etwa 10 Minuten der Goldpartikel wie in Punkt b) oder b') erhalten durchgeführt wird, die in einem Polyacrylamidgelladungspuffer, umfassend SDS, suspendiert sind.

**14.** Verfahren nach einem der Ansprüche 1 bis 13, wobei die Polyacrylamidgradient ein 4-20 % Gradient ist.

**Revendications**

**1.** Procédé pour aider au diagnostic précoce de l'adénocarcinome pancréatique qui permet d'identifier des sujets à un stade précoce de la maladie ou à risque d'adénocarcinome pancréatique pour lesquels il est nécessaire ou opportun de réaliser des examens de second niveau en classant un échantillon de plasma de ces sujets selon deux ou plusieurs niveaux de risque dans lesquels la classification dudit échantillon dans au moins un desdits niveaux de risque indiquant la nécessité ou l'opportunité de réaliser des examens de second niveau, comprenant les étapes suivantes :

a) la fourniture d'un échantillon de plasma à partir d'un échantillon de sang d'un sujet à analyser ;

b) l'incubation dudit échantillon de plasma avec des nanoparticules d'or de manière à permettre la formation d'une couronne protéique sur lesdites nanoparticules, ledit passage b) étant éventuellement suivi d'un passage b') dans lequel le matériau incubé est soumis à une centrifugation et à un ou plusieurs lavages avec un tampon phosphate afin d'éliminer des protéines faiblement liées ;

c) la séparation des protéines qui composent ladite couronne protéique des nanoparticules ;

d) la soumission desdites protéines à une électrophorèse sur un gel dénaturant à gradient de polyacrylamide afin d'obtenir le profil protéique (Pp) de la couronne protéique obtenue aux points b) ou b') ;

e) la fourniture d'au moins une fonction discriminante ($f(\alpha_1, \alpha_2,... \alpha_n)$, $g(\alpha_1, \alpha_2, \alpha_3,... \alpha_n)$) et deux bandes ou plus de poids moléculaire ($A_1$, $A_2$... $A_n$), ladite au moins une fonction discriminante ($f(\alpha_1, \alpha_2,... \alpha_n)$, $g(\alpha_1, \alpha_2, \alpha_3,... \alpha_n)$) et lesdites bandes de poids moléculaire ($A_1$, $A_2$, .... $A_n$) étant prédéterminé sur la base d'un ensemble d'échantillons de référence ;

f) le calcul d'une valeur intégrale de surface ($VA_1$, $VA_2$... $VA_n$) dudit profil protéique (Pp) pour chacune desdites bandes de poids moléculaire ($A_1$, $A_2$... $A_n$) ;

g) le calcul d'au moins une valeur discriminante ($VD_f$, $VD_g$) de ladite au moins une fonction discriminante ($f(\alpha_1, \alpha_2,... \alpha_n)$, $g(\alpha_1, \alpha_2, \alpha_3,... \alpha_n)$) pour au moins une paire desdites valeurs de surface intégrale ($VA_1$, $VA_2$... $VA_n$) ;

h) le classement de l'échantillon de plasma en comparant ladite au moins une valeur discriminante ($VD_f$, $VD_g$) à au moins une valeur seuil prédéfinie (VS).

2. Procédé selon la revendication 1, dans lequel lesdits examens de second niveau comprennent un ou plusieurs examens parmi : une tomographie assistée par ordinateur de l'abdomen, une IRM de l'abdomen, une échoendoscopie, une CPRE.

3. Procédé selon les revendications 1 ou 2, dans lequel ladite au moins une fonction discriminante ($f(\alpha_1, \alpha_2,... \alpha_n)$, $g(\alpha_1, \alpha_2, \alpha_3,... \alpha_n)$) est obtenue par analyse de discrimination linéaire.

4. Procédé selon l'une quelconque des revendications précédentes, dans lequel ladite au moins une fonction discriminante ($f(\alpha_1, \alpha_2, ... \alpha_n)$, $g(\alpha_1, \alpha_2, \alpha_3, ... \alpha_n)$) comprend une première fonction ($f(\alpha_1, \alpha_2)$) représentée par l'équation d'une ligne droite du type :

$$f(\alpha_1, \alpha_2) = r1 + r2 * \alpha_1 + r3 * \alpha_2 = 0$$

où r1, r2 et r3 sont les coefficients de la ligne droite et $\alpha_1$ et $\alpha_2$ sont les valeurs intégrales de surface.

5. Procédé selon l'une quelconque des revendications 1 à 4, dans lequel ladite au moins une fonction discriminante ($f(\alpha_1, \alpha_2,...\alpha_n)$, $g(\alpha_1, \alpha_2, \alpha_3,...\alpha_n)$) comprend une seconde fonction ($g(\alpha_1, \alpha_3, \alpha_4)$) représentée par l'équation d'un plan du type :

$$g(\alpha_1, \alpha_3, \alpha_4) = p1 + p2 * \alpha_1 + p3 * \alpha_3 + p4 * \alpha_4 = 0$$

où p1, p2, p3 et p4 sont des coefficients du plan et $\alpha_1$, $\alpha_3$ and $\alpha_4$ sont des valeurs intégrales de surface.

6. Procédé selon l'une quelconque des revendications précédentes, dans lequel lesdites deux bandes ou plus de poids moléculaire ($A_1$, $A_2$... $A_n$) sont déterminés de manière à ce que chaque pic - correspondant à une bande électrophorétique respective - de chaque profil protéique de l'ensemble d'échantillons de référence appartienne à une et unique desdites bandes.

7. Procédé selon l'une quelconque des revendications 1 à 6, dans lequel ladite au moins une valeur seuil prédéfinie (VS) est égale à zéro et ledit échantillon de plasma est classé comme étant à risque si la valeur discriminante ($VD_f$, $VD_g$) est supérieure à zéro.

8. Procédé selon l'une quelconque des revendications 1 à 7, dans lequel lesdites bandes de poids moléculaire peuvent être, par exemple, deux ou plus parmi $A_1$ : 10 à 20 kDa ; $A_2$ : 20 à 25 kDa ; $A_3$ : 25 à 35 kDa ; $A_4$ : 35 à 45 kDa.

9. Procédé selon l'une quelconque des revendications 1 à 7, lesdites nanoparticules d'or ont un diamètre moyen de 100 nm.

**10.** Procédé selon l'une quelconque des revendications 1 à 9, l'étape b) est réalisée à une température comprise entre 35 et 40 °C pendant une durée comprise entre 40 et 120 minutes.

**11.** Procédé selon la revendication 10, dans lequel ladite température est de 37° C et/ou ladite durée est de 55 à 56 minutes.

**12.** Procédé selon l'une quelconque des revendications 1 à 11, dans lequel ladite étape b) est suivie d'une étape b') dans laquelle le matériel incubé est soumis à une centrifugation et à un ou plusieurs lavages avec un tampon phosphate pour éliminer des protéines faiblement liées.

**13.** Procédé selon l'une quelconque des revendications 1 à 12, dans lequel ladite séparation au point c) est réalisée en faisant bouillir pendant environ 10 minutes les particules d'or comme obtenues au point b) ou b') suspendues dans un tampon de charge de gel de polyacrylamide comprenant du SDS.

**14.** Procédé selon l'une quelconque des revendications 1 à 13, dans lequel ledit gradient de polyacrylamide est un gradient de 4 à 20 %.

**FIG. 1**

**FIG. 2a**

**Fig. 2b**

**FIG. 2c**

**FIG. 2d**

**FIG. 3a**

**FIG. 3b**

FIG. 3c

**FIG. 4**

**FIG. 5**

# EP 4 004 941 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **YACHIDA S ; JONES S ; BOZIC I et al.** Distant metastasis occurs late during the genetic evolution of pancreatic cancer.. *Nature*, 2010, vol. 467 (7319), 1114-7
- **HAJIPOUR, MOHAMMAD JAVAD et al.** Personalized disease-specific protein corona influences the therapeutic impact of graphene oxide. *Nanoscale*, 2015, vol. 7 (19), 8978-8994
- **COLAPICCHIONI et al.** Personalized liposome-protein corona in the blood of breast, gastric and pancreatic cancer patients. *International Journal of Biochemistry and Cell Biology*, 2016, vol. 75, 180-187
- **CAPUTO et al.** A protein corona-enabled blood test for early cancer detection. *Nanoscale*, 2017, vol. 9, 349-354